# EUROPEAN PATENT APPLICATION

(11) **EP 4 082 660 A1**
(43) Date of publication of application: **02.11.2022**
(21) Application number: 22164290.3
(22) Date of filing: 25.03.2022
(51) Int. Cl.: B01J 35/00, B01J 37/03

(54) **PHOTOCATALYST AND METHOD FOR FABRICATING THE SAME**

(30) Priority: 26.04.2021 US 202163201347 P; 17.03.2022 US 202217655181
(71) Applicant: Nano and Advanced Materials Institute Limited, Shatin, New Territories (HK)
(72) Inventor: KEUNG, Lok Hang, Shatin, New Territories (HK); CAI, Juanfang, Shatin, New Territories (HK); SO, King Ho, Shatin, New Territories (HK); YEE, Ka Kit, Shatin, New Territories (HK); LEE, Ka Chun, Shatin, New Territories (HK); LAI, Lok Shun, Shatin, New Territories (HK)
(74) Representative: Fabry, Bernd

(57) **Abstract**

The present invention provides a metal/metal oxide doped-WO₃ flower-like assemblies as a photocatalyst applied to photocatalytic inactivation of influenza virus and bacteria under UV or visible light activation, and further provides a surface-modulator-driven synthesis method for producing WO3 flower-like assemblies, as well as doping methods for doping the metal/metal oxide to the WO3 flower-like assemblies. The metal/metal oxide doped in WO3 flower-like assemblies can further enhance the antiviral and antibacterial performances.

## Description

### TECHINCAL FIELD

The present invention relates to a photocatalyst and a method for fabricating the same.

### BACKGROUND

Pathogenic microorganisms such as bacteria, viruses and fungi are the major concern for human's health because they severely harm to the body and become lethal. In spite of the remarkable research and development in the treatment and prevention procedures, infectious disease still remains the major cause of death in the world. Antimicrobial have been used for many years in controlling the growth of pathogenic organisms. However, long time usage of antimicrobial led to develop resistant microbes. Hence, new, safe and effective disinfectants are needed to overcome problems associated with pathogenic microorganisms.

Photocatalytic oxidation (PCO) catalyst is an alternative remediation technology, which offers a number of advantages over conventional disinfectant. PCO catalyst can degrade a broad range of pollutants into harmless products such as CO2 and H₂O under irradiation. Under irradiation of light, reactive oxygen species (ROS) such as hydroxyl radicals (·OH) and super oxide anion (O₂⁻·) will be generated. These ROS are highly useful in killing bacteria and virus. Titanium dioxide (TiO₂) is by far the most investigated photocatalyst providing great antiviral performance.

Nevertheless, TiO₂ responds only to UV light region, and shows insufficient effect under practical illuminance or indoor environment. Especially, ultraviolet light required for excitation of an antibacterial agent based on titanium oxide is limited and its illuminance is low in the interior of a general house, so that the irradiation of light sufficient for the product to exert the antibacterial performance cannot be obtained.

Compared with TiO₂, tungsten oxide (WO₃) provides desirable properties such as chemically stable, visible light active, and high earth abundance. Particularly, WO₃ can be activated by visible light or UV light source to generate ROS for providing the antiviral and antibacterial functions.

Although WO₃ is an attractive candidate to function as visible light active PCO, the traditional hydrothermal method for preparing WO₃ requires high pressure and relatively high temperature (180°C). In addition, the conventional WO₃ nanostructures, such as nanoparticles and nanospheres, exhibit relatively low photocatalytic activity in view of the actual applications.

A need therefore exists for a novel photocatalyst that eliminates or at least diminishes the disadvantages and problems described above.

### SUMMARY OF THE INVENTION

Provided herein is a photocatalyst comprising: a tungsten oxide (WO₃) flower-like assembly comprising corners and the edges at the surface of the WO₃ flower-like assembly; and particles of a metal or a metal oxide; wherein the particles are distributed on the surface of the WO₃ flower-like assembly.

In certain embodiments, wherein the WO₃ flower-like assembly comprises WO₃ nanoplates aggregated to form at least a portion of the surface of the WO₃ flower-like assembly for providing the corners and the edges.

In certain embodiments, the WO₃ flower-like assembly comprises WO₃ nanoplates aggregated to form the surface of the WO₃ flower-like assembly for providing the corners and the edges.

In certain embodiments, the metal is titanium (Ti), zirconium (Zr), manganese (Mn), iron (Fe), palladium (Pd), platinum (Pt), copper (Cu), silver (Ag), zinc (Zn), aluminum (Al) or cerium (Ce); and the metal oxide is an oxide of Ti, Zr, Mn, Fe, Pd, Pt, Cu, Ag, Zn, Al or Ce.

In certain embodiments, each particle has a particle size between 100 nm and 900 nm.

In certain embodiments, the photocatalyst has a mass concentration of the particles between 0.01% and 50%.

In certain embodiments, the WO₃ flower-like assembly has a particle size between 100 nm and 250 nm, and has a round shape or a spherical shape.

In certain embodiments, the WO₃ flower-like assembly is further doped with a metal ion, the metal ion being an ion of Ti, Zr, Mn, Fe, Pd, Pt, Cu, Ag, Zn, Al or Ce.

Provided herein is a disinfection material comprising the photocatalyst described above.

Provided herein is a method for fabricating a photocatalyst comprising: providing a modulator solution comprising a dicarboxylic acid or oxalic acid; providing a sodium tungstate dihydrate solution; mixing the sodium tungstate dihydrate solution and the modulator solution thereby forming a first mixture solution; adding an acid into the first mixture solution thereby forming a second mixture solution; heating the second mixture solution thereby forming a first precipitate; collecting the first precipitate from the second mixture solution; drying the first precipitate thereby forming WO₃ flower-like assemblies; and doping the WO₃ flower-like assemblies with particles of a metal or a metal oxide such that the particles are distributed on the surface of each WO₃ flower-like assembly thereby forming the photocatalyst.

In certain embodiments, the step of doping comprises mechanically mixing the WO₃ flower-like assemblies and a powder comprising the particles to distribute the particles on the surface of each WO₃ flower-like assembly.

In certain embodiments, the step of doping comprises: dispersing the WO₃ flower-like assemblies in a solution containing the particles to distribute the particles on the surface of each WO₃ flower-like assembly thereby forming second precipitate; collecting the second precipitate from the solution; and drying the second precipitates thereby forming the photocatalyst.

In certain embodiments, the metal is Ti, Zr, Mn, Fe, Pd, Pt, Cu, Ag, Zn, Al or Ce.

In certain embodiments, the metal oxide is an oxide of Ti, Zr, Mn, Fe, Pd, Pt, Cu, Ag, Zn, Al or Ce.

In certain embodiments, the dicarboxylic acid is 1,4-benzendicarboxylic acid.

In certain embodiments, the sodium tungstate dihydrate solution has a concentration of sodium tungstate dihydrate between 0.05 mol/L and 0.30 mol/L.

In certain embodiments, the mass ratio of sodium tungstate dihydrate and oxalic acid in the first mixture solution is between 1:1.1 and 1:2.0.

In certain embodiments, the acid is hydrochloric acid (HCl).

In certain embodiments, the HCl is added to adjust the pH value of the second mixture solution to be 0.1 to 1.0.

In certain embodiments, the second mixture solution is heated at 80-100°C for 8-12 hours.

This summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter. Other aspects of the present invention are disclosed as illustrated by the embodiments hereinafter.

### BRIEF DESCRIPTION OF DRAWINGS

The appended drawings, where like reference numerals refer to identical or functionally similar elements, contain figures of certain embodiments to further illustrate and clarify the above and other aspects, advantages and features of the present invention. It will be appreciated that these drawings depict embodiments of the invention and are not intended to limit its scope. The invention will be described and explained with additional specificity and detail through the use of the accompanying drawings in which:
Figure 1A depicts a method for fabricating metal oxide-doped WO₃ flower-like assemblies according to certain embodiments;
Figure 1B depicts the ROS formation mechanism by the metal oxide-doped WO₃ flower-like assemblies;
Figure 2A shows a field emission scanning microscopy (FESEM) image of WO₃ flower-like assemblies of Example 1 (with H₂C₂O₄:Na₂WO₄ = 1.4:1) with low magnification;
Figure 2B shows a FESEM image of the WO₃ flower-like assemblies high magnification;
Figure 3A shows a FESEM image of CuO-doped WO₃ nanoflowers of Example 2;
Figure 3B shows the energy-dispersive X-ray spectroscopy (EDX) of the CuO-doped WO₃ nanoflowers;
Figure 4 shows a schematic diagram depicting an irradiation set-up for an antibacterial test;
Figure 5A shows an antibacterial test *(E. coli)* result of a control glass slide sample without WO₃ coating;
Figure 5B shows an antibacterial test *(E. coli)* result of a WO₃ flower-like assemblies-coated sample prepared by Example 1;
Figure 6A shows an antibacterial test *(E. coli)* result of a control glass slide sample without WO₃ coating;
Figure 6B shows an antibacterial test *(E. coli)* result of a commercial WO₃ particles-coated sample;
Figure 6C shows an antibacterial test *(E. coli)* result of a copper(II) oxide (CuO)-doped WO₃ flower-like assemblies-coated sample prepared by Example 2;
Figure 6D shows a FESEM image of the commercial WO₃ particles; and
Figure 6E shows a FESEM image of the CuO-doped WO₃ flower-like assemblies.

Skilled artisans will appreciate that elements in the figures are illustrated for simplicity and clarity and have not necessarily been depicted to scale.

### DETAILED DESCRIPTION OF THE INVENTION

Accordingly, the present disclosure provides a metal/metal oxide-doped WO₃ flower-like assemblies as a photocatalyst for antiviral and antibacterial functions and a surface-modulator-driven synthesis method for fabricating the WO₃ flower-like assemblies. The metal/metal oxide-doped WO₃ flower-like assemblies can be applied to photocatalytic inactivation of influenza virus and bacteria under UV or visible light activation. The metal/metal oxide doped in WO₃ flower-like assemblies can further enhance the antiviral and antibacterial performance.

As the morphology of WO₃ affects the charge separation and recombination during the photocatalytic process. The present WO₃ flower-like assembly resembles a flower and is assembled by nanostructures (i.e., nano-building-blocks) aggregated to form the WO₃ flower-like assembly resembles. The WO₃ flower-like assembly includes corners and edges, and atoms located in corners and edges have more catalytic activity because of the low coordination numbers. Particularly, the WO₃ flower-like assembly includes WO₃ nanoplates aggregated to form the surface of the WO₃ flower-like assembly. As the WO₃ nanoplates have sharp corners and edges, they shows higher photocatalytic activity in comparison with the WO₃ nanospheres. In addition, the flower shape of the WO₃ flower-like assembly can further increase the surface area for enhancing the antiviral and antibacterial performance.

Provided herein is a photocatalyst comprising: a tungsten oxide (WO₃) flower-like assembly comprising corners and the edges at the surface of the WO₃ flower-like assembly; and particles of a metal or a metal oxide; wherein the particles are distributed on the surface of the WO₃ flower-like assembly.

In certain embodiments, the particles are distributed partially or fully on the surface of each WO₃ flower-like assembly.

In certain embodiments, the WO₃ flower-like assembly comprises WO₃ nanoplates aggregated to form at least a portion of the surface of the WO₃ flower-like assembly for providing the corners and the edges.

In certain embodiments, the WO₃ flower-like assembly comprises WO₃ nanoplates aggregated to form the surface of the WO₃ flower-like assembly for providing the corners and the edges.

In certain embodiments, the metal is titanium (Ti), zirconium (Zr), manganese (Mn), iron (Fe), palladium (Pd), platinum (Pt), copper (Cu), silver (Ag), zinc (Zn), aluminum (Al) or cerium (Ce); and the metal oxide is titanium oxide, zirconium oxide, manganese oxide, iron oxide, palladium oxide, platinum oxide, copper oxide, silver oxide, zinc oxide, aluminum oxide or cerium oxide.

In certain embodiments, each particle has a particle size between 100 nm and 900 nm

In certain embodiments, the photocatalyst has a mass concentration of the particles between 0.01% and 50%.

In certain embodiments, the WO₃ flower-like assembly has a particle size between 100 nm and 250 nm.

In certain embodiments, the WO₃ flower-like assembly has a round shape or a spherical shape.

In certain embodiments, the WO₃ flower-like assembly is further doped with a metal ion, the metal ion being an ion of Ti, Zr, Mn, Fe, Pd, Pt, Cu, Ag, Zn, Al or Ce.

Provided herein is a disinfection material comprising the photocatalyst described above.

Provided herein is a method for fabricating a photocatalyst comprising: providing a modulator solution comprising a dicarboxylic acid or oxalic acid; providing a sodium tungstate dihydrate solution; mixing the sodium tungstate dihydrate solution and the modulator solution thereby forming a first mixture solution; adding an acid into the first mixture solution thereby forming a second mixture solution; heating the second mixture solution thereby forming a first precipitate; collecting the first precipitate from the second mixture solution; drying the first precipitate thereby forming WO₃ flower-like assemblies; and doping the WO₃ flower-like assemblies with particles of a metal or a metal oxide such that the particles are distributed on the surface of each WO₃ flower-like assembly thereby forming the photocatalyst.

In certain embodiments, the particles are distributed partially or fully on the surface of each WO₃ flower-like assembly.

In certain embodiments, the step of doping comprises mechanically mixing the WO₃ flower-like assemblies and a powder comprising the particles to distribute the particles on the surface of each WO₃ flower-like assembly.

In certain embodiments, the step of doping comprises: dispersing the WO₃ flower-like assemblies in a solution containing the particles to distribute the particles on the surface of each WO₃ flower-like assembly thereby forming second precipitate; collecting the second precipitate from the solution; and drying the second precipitates thereby forming the photocatalyst.

In certain embodiments, the metal is Ti, Zr, Mn, Fe, Pd, Pt, Cu, Ag, Zn, Al or Ce.

In certain embodiments, the metal oxide is titanium oxide, zirconium oxide, manganese oxide, iron oxide, palladium oxide, platinum oxide, copper oxide, silver oxide, zinc oxide, aluminum oxide or cerium oxide.

In certain embodiments, the dicarboxylic acid is 1,4-benzendicarboxylic acid.

In certain embodiments, the sodium tungstate dihydrate solution has a concentration of sodium tungstate dihydrate between 0.05 mol/L and 0.30 mol/L.

In certain embodiments, the mass ratio of sodium tungstate dihydrate and oxalic acid in the first mixture solution is between 1:1.1 and 1:2.0.

In certain embodiments, the acid is hydrochloric acid (HCl)

In certain embodiments, the HCl is added to adjust the pH value of the second mixture solution to be 0.1 to 1.0.

In certain embodiments, the second mixture solution is heated at 80-100°C for 8-12 hours.

In certain embodiments, a method for fabricating a photocatalyst comprises: preparing a water solution of modulator by dissolving oxalic acid powder in deionized water; preparing a water solution of sodium tungstate dihydrate powder by dissolving sodium tungstate dihydrate powder in deionized water; dropwise addition of the sodium tungstate dihydrate water solution to the modulator water solution to form a first mixture solution; adding 37% HCl to the first mixture solution to from a second mixture solution with the pH value between 0.1 and 1.0; heating and stirring the second mixture solution; collecting the precipitate from the second mixture solution by centrifugation; washing the precipitate with water and ethanol; and air-drying the solid sample; and doping the solid sample with metal oxide.

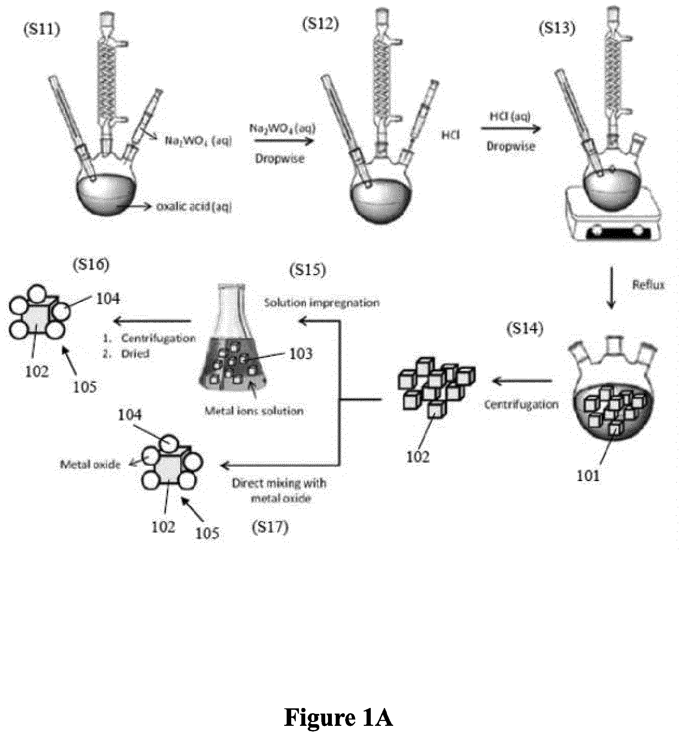

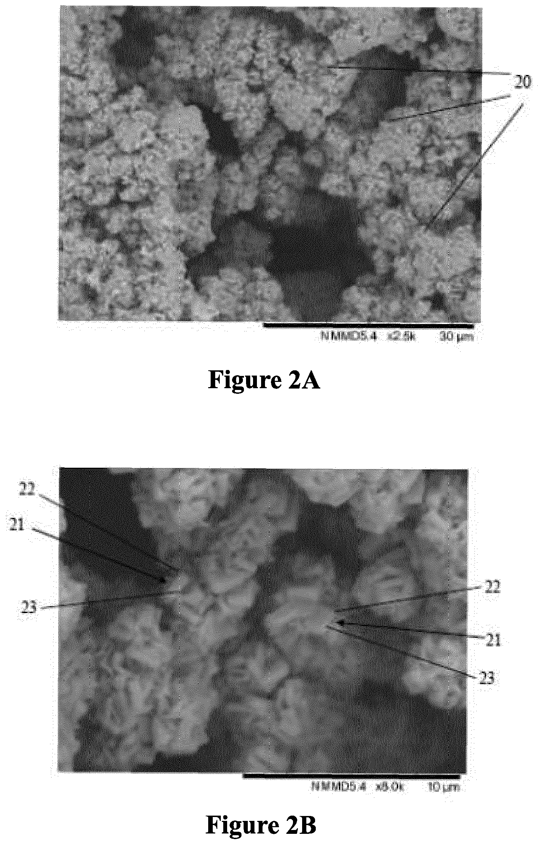

The present method for fabricating WO₃ flower-like assemblies is a simple surface-modulator-driven synthesis method. As shown Figure 1A, the WO₃ flower-like assemblies of the certain embodiments are prepared by using sodium tungstate dihydrate as a starting material, oxalic acid as a modulator for mediating the structure of the synthesized WO₃ (e.g., assisting the formation of monoclinic), and HCl to control the pH value of the precursor aqueous solution. HCl is preferably used since alternate acid may lead to formation of by-products. The oxalic acid acts as a modulator to control the growth direction of WO₃ nanoparticles for synthesizing self-assembled flower-like structure/nanoflowers. The introduction of enough amount of oxalic acid (e.g., oxalic acid/sodium tungstate dihydrate ratio of 1.5:1) provides sufficient oxalate ions to form hydrogen bonds with structural water of WO₃ nanoplates, which results in the aggregation of nanoplates, and appears in a flower-like assemblies morphology (e.g., as shown in Figures 2A and 2B).

Referring to Figure 1A, in step S11, sodium tungstate dihydrate solution is added into the oxalic acid by dropwise to form a first mixture solution. In step S12, HCl is added into the first mixture solution by dropwise to form a second mixture solution. In step S13, the second mixture solution is heated and refluxed to form a precipitate 101 of WO₃ flower-like assemblies. In step S 14, the precipitate 101 is collected by centrifugation and dried to form dry WO₃ flower-like assemblies 102. In step S15, the dry WO₃ flower-like assemblies 102 are dispersed in a metal ion solution for doping the WO₃ flower-like assemblies with metal oxide particles 104 to form a precipitate 103 of metal oxide-doped WO₃ flower-like assemblies. In this embodiment, the metal ions (e.g., Ti, Zr, Mn, Fe, Pd, Pt, Cu, Ag, Zn, Al or Ce ions) in the metal ion solution also act as a dopant doped into the WO₃ flower-like assemblies. In step S16, the precipitate 103 is collected by centrifugation and dried to form dry metal oxide-doped WO₃ flower-like assemblies 105, and each dry metal oxide-doped WO₃ flower-like assembly includes the dry WO₃ flower-like assembly 102 and the metal oxide particles 104 distributed on the dry WO₃ flower-like assembly 102. Alternatively, after the step S14, the dry WO₃ flower-like assemblies 102 are directly and mechanically mixed with a powder having the metal oxide particles 104 to form the metal oxide-doped WO₃ flower-like assemblies 105 as shown in step S17.

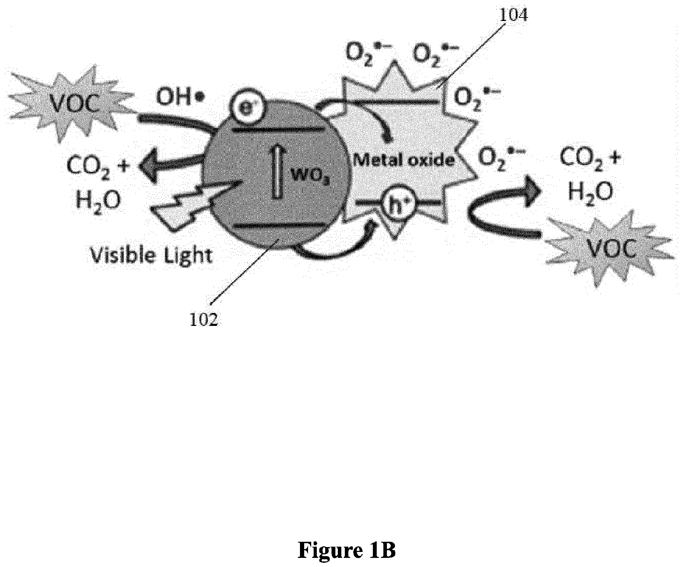

The metal oxide particles 104 can enhance the photocatalytic activity of WO₃ flower-like assembly 102. These co-catalysts can facilitate the multiple-electron reduction of oxygen, then the electron on the WO₃ conduction band reduces oxygen to super oxide anion O2-· effectively as shown in Figure 1B. The formation of ROS is important for killing bacteria, virus, as well as organic pollutants dissociation.

### Example 1: Preparation of WO₃ flower-like assemblies

As a specific example of the WO₃ flower-like assemblies, sodium tungstate dihydrate (3.3g, 0.01mmol) was dissolved in 60 mL of deionized water to get a transparent solution of 0.17mol/L. Oxalic acid (1.5g, 0.015mmol) was subsequently added into the reaction system and the obtain solution was stirred at room temperature for 30 mins. 1.5 mL of 37% HCl aqueous solution was added dropwise into the above solution under continuous stirring until pH 0.1. The reaction vessel was transferred into an oil bath of 90°C. After 4 hours reaction, the final yellow precipitates were centrifuged and washed repeatedly with deionized water and ethanol, followed by air-drying the sample in air, thus WO₃ flower-like assemblies were obtained.

The SEM image of Figure 2A shows a plurality of WO₃ flower-like assemblies 20 of Example 1. As shown in Figure 2B, the WO₃ flower-like assembly 20 have a plurality of WO₃ nanoplates 21 aggregated randomly to form the surface of the WO₃ flower-like assembly 20, and the WO₃ nanoplate 21 has corners 22 and edges 23, and atoms located in the corners 22 and the edges 23 can provide more catalytic activity for enhancing the antibacterial and antiviral performances.

### Example 2: Preparation of CuO-doped WO₃ flower-like assemblies

As a specific example of the CuO-doped WO₃ flower-like assemblies, there is a mixed powder containing 99.5 wt% of WO₃ flower-like assemblies and 0.5 wt% of copper (II) oxide powder. The mixed powder is prepared by grounding copper (II) oxide and WO₃ flower-like assemblies together for 10 mins.

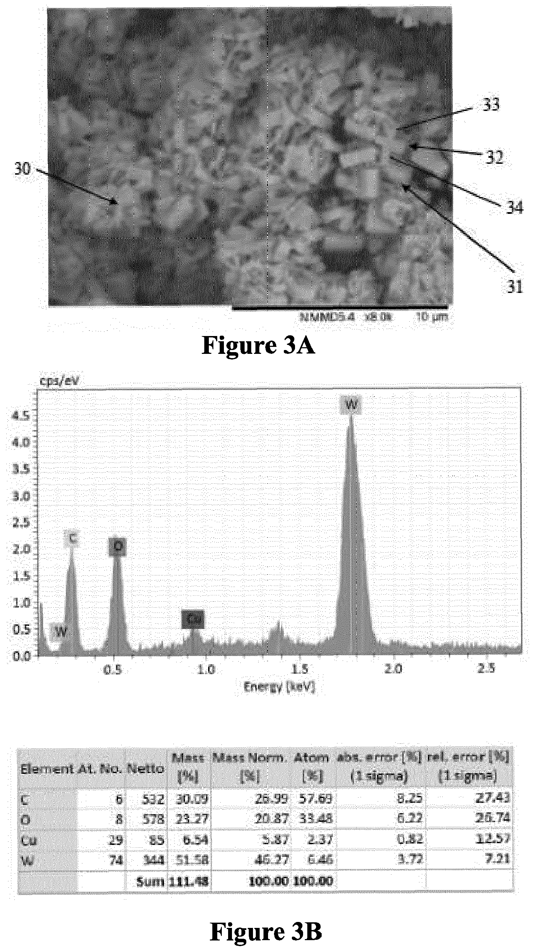

Figure 3A shows CuO-doped WO₃ nanoflower 30 of Example 2 including WO₃ nanoflower 31 and CuO particles distributed on the surface of the WO₃ nanoflower 31. The WO₃ nanoflower 31 includes WO₃ nanoplates 32 aggregated randomly to form the surface of the WO₃ nanoflower 31 and the WO₃ nanoplate 31 has corners 33 and edges 34. Figure 3B shows the EDX of the CuO-doped WO₃ nanoflowers 30 which indicates the presence of CuO, and the CuO-doped WO₃ nanoflowers has a mass concentration of Cu with 6.54%.

To evaluate the antibacterial and antivirus properties, the WO₃ flower-like assemblies of Example 1 were undergone an antibacterial property evaluation test adopted from ISO 27447:2009 and an antiviral property evaluation test adopted from ISO18184:2019.

To prepare the test specimens, the WO₃ flower-like assemblies were mixed with a dispersion medium e.g., water. A dispersion process was performed by an ultrasonic disperser. A testing sample was produced by coating the obtained dispersion liquid on a test piece such as a glass plate. This general method includes dripping, spin coating, dipping, spraying or the like. The WO₃ flower-like assemblies were adhered in a range of 0.02 mg/cm² or more and 40 mg/cm² or less on a 5 × 5 cm glass slide.

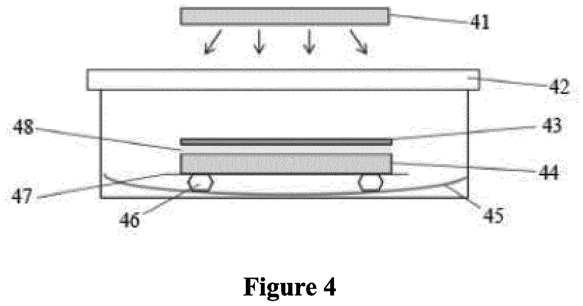

Figure 4 shows a schematic diagram depicting an irradiation set-up for the antibacterial test. The irradiation set-up includes light source 41, plastic lid 42, cover slide glass 43, WO₃-coated glass slide 44, wet filter paper 45, metal nut 46, metal plate 47, and bacterial suspension 48.

The percent reduction of bacteria was evaluated according to a glass adhesion test based on an adopted ISO 27447:2009 test (replacing UV light source to a visible light source 41). The bacterial suspension 48 (0.1 mL; 10⁵ CFU mL⁻¹) was pipetted onto the test surface (5×5 cm²) and a cover glass applied (4 × 4 mm²). The sample was placed in a square Petri dish (100 × 100 × 20 mm³) equipped with wet filter paper 45 and a square metal plate 47 for support as shown in Figure 4. At least one bacterium selected from among Staphylococcus aureus, Escherichia coli, Klebsiella pneumonia and Pseudomonas aeruginosa. A visible light lamp (1000 lx) was used as a light source. After irradiation for 4 hours, sample on the cover glass were shaken out into 3 mL of saline solution, serially diluted and spread onto an agar media. In order to determine the number of viable cells (counted as CFU mL⁻¹), the samples were incubated at 37 °C for 24 h and the bacterial colonies were then counted.

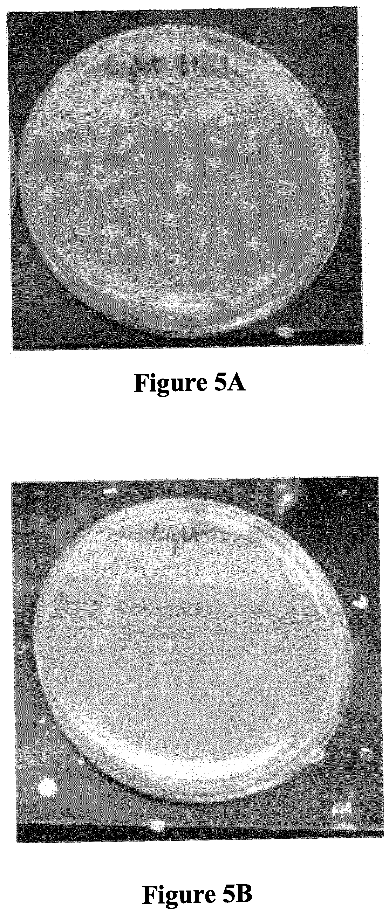

Figures 5A and 5B shows the antibacterial test results of the control sample and WO₃ flower-like assemblies on LB agar plate. As shown in Figure 5A, E.coli colonies is visible on the control sample while no visible colonies appears on the WO₃ flower-like assemblies-treated sample as shown in Figure 5B, which indicates the WO₃ flower-like assemblies can kill bacteria effectively.

The antiviral activity of the WO₃ flower-like assemblies was evaluated according to an adopted ISO18184:2019 test method (using WO₃ flower-like assemblies coated glass plates as test specimen and irradiating the sample with visible light). A visible light source (1000 lx) was used and the sample was irradiated for 4 hours. The concentrated virus was diluted with PBS to adjust the virus titer at six TCID₅₀/ml. The reaction was carried out at room temperature (25°C). The WO₃ flower-like assemblies coated glass plate was also evaluated in a dark condition in a stainless steel box. At the indicated time, the virus spotted glass plate was taken and rinsed with 1ml of maintenance medium for MDCK cells in a plastic bag. The recovered virus was transferred into a microtube and centrifuged to remove debree, and the resulting supernatant was titrated on MDCK cells.

The percent reduction of bacteria or virus was calculated as follows: Percent Reduction = (A-B)×100/A, where A is the number of viable microorganisms before treatment, and B is the number of viable microorganisms after treatment.

Table 1 below shows test results of the sample of CuO-doped WO₃ flower-like assemblies of Example 2 according to antiviral test (H1N1). The sample of CuO-doped WO₃ flower-like assemblies show 99.99% of virus reduction.

**Table 1:**

| **Virus** | **Irradi ation time (h)** | **Testin g condit ion** | **Gro up** | **First test Log(TCI D₅₀/ml)** | **Second test Log(TCI D₅₀/ml)** | **Third test Log(TCI D₅₀/ml)** | **Average value Log(TCI D₅₀/ml)** | **Mean logarit hm of inactiv ation** | **Antiv iral efficie ncy (%)** |
|---|---|---|---|---|---|---|---|---|---|
| A/PR 8/34 (H1N 1) | 4 | Irradia tion condit ion | Cont rol sam ple | 5.67 | 6.00 | 6.00 | 5.89 | 5.89 | >99.9 9 |
| | | | Test sam ple | 0.00 | 0.00 | 0.00 | 0.00 | | |
| | | Dark condit ion | Cont rol sam ple | 5.67 | 6.00 | 6.00 | 5.89 | 5.89 | >99.9 9 |
| | | | Test sam ple | 0.00 | 0.00 | 0.00 | 0.00 | | |

The antibacterial and antivirus properties of WO₃ flower-like assemblies can be enhanced by doping with transition metal element or their oxides. The method of combining the WO₃ flower-like assemblies and the transition metal element (specifically, a single element or a metal oxide of at least one element selected from among Ti, Zr, Mn, Fe, Pd, Pt, Cu, Ag, Zn, Al and Ce) includes various combining methods such as a direct mixing method for mixing powders and an impregnation method.

To evaluate the antibacterial properties, the CuO-doped WO₃ flower-like assemblies of Example 2 were undergone an antibacterial property evaluation test adopted from ISO 27447:2009.

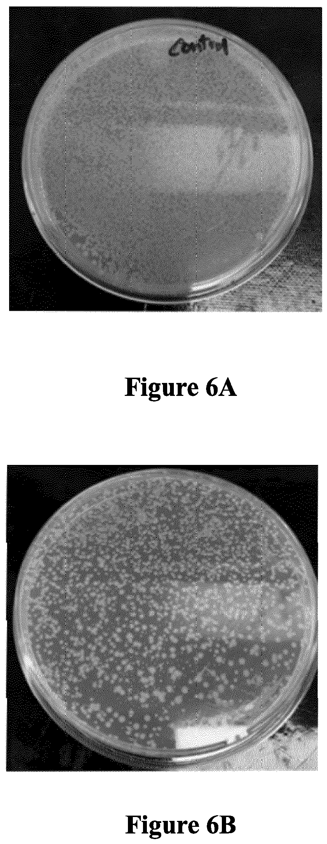

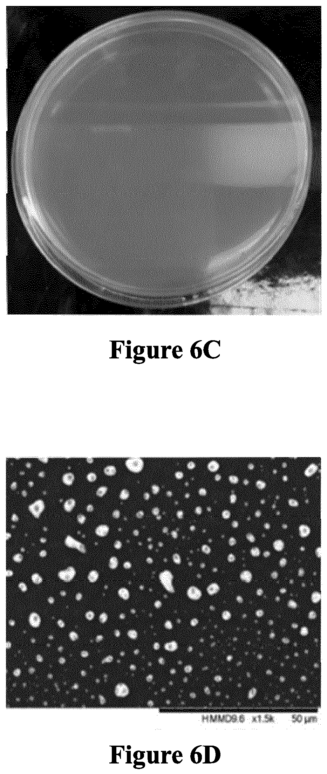

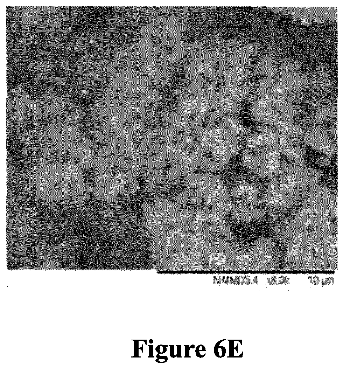

Figures 6A-6C show the antibacterial test results of the control, commercial WO₃ particles and WO₃ flower-like assemblies on LB agar plate. As shown in Figures 6A and 6B, *E. coli* colonies are visible on the control sample and the commercial WO₃ particles-coated sample while no visible colonies appears on the CuO-doped WO₃ flower-like assemblies-coated sample as shown in Figure 6C, which indicates the CuO-doped WO₃ flower-like assemblies can kill bacteria effectively. Correspondingly, the morphology of the commercial WO₃ particles and CuO-doped WO₃ flower-like assemblies are shown in Figures 6D and 6E respectively. In addition, some reports revealed that Cu(II) doped WO₃ nanoparticles merely provide -85% of antibacterial reduction which is smaller when compared with CuO-doped WO₃ flower-like assemblies with >99.99% under 120 mins visible light irradiation. It indicates that the structure/morphology of the WO₃ is a key factor in antibacterial/antiviral property.

Thus, it can be seen that an improved photocatalyst exerts the antibacterial and antiviral performances without requiring irradiation of UV light. Therefore, practical antibacterial performance can be obtained even when the metal/metal oxide-doped WO₃ flower-like assemblies catalyst is applied to products, which are used in an indoor environment having a low illuminance, such as ceilings, walls, floors, furniture and home electric appliances in the interior. In addition, metal/metal oxide doped in WO₃ flower-like assemblies can enhance the antibacterial and antiviral performances.

Although the invention has been described in terms of certain embodiments, other embodiments apparent to those of ordinary skill in the art are also within the scope of this invention. Accordingly, the scope of the invention is intended to be defined only by the claims which follow.

## Claims

1. A photocatalyst comprising:
a tungsten oxide (WO₃) flower-like assembly comprising corners and edges at a surface of the WO₃ flower-like assembly; and
particles of a metal or a metal oxide,
wherein the particles are distributed on the surface of the WO₃ flower-like assembly.

2. The photocatalyst of claim 1, wherein the WO₃ flower-like assembly comprises WO₃ nanoplates aggregated to form at least a portion of the surface of the WO₃ flower-like assembly for providing the corners and the edges.

3. The photocatalyst of claim 1, wherein the WO₃ flower-like assembly comprises WO₃ nanoplates aggregated to form the surface of the WO₃ flower-like assembly for providing the corners and the edges.

4. The photocatalyst of claim 1, wherein the metal is titanium (Ti), zirconium (Zr), manganese (Mn), iron (Fe), palladium (Pd), platinum (Pt), copper (Cu), silver (Ag), zinc (Zn), aluminum (Al) or cerium (Ce); and the metal oxide is an oxide of Ti, Zr, Mn, Fe, Pd, Pt, Cu, Ag, Zn, Al or Ce.

5. The photocatalyst of claim 1, wherein each particle has a particle size between 100 nm and 900 nm.

6. The photocatalyst of claim 1, wherein the photocatalyst has a mass concentration of the particles between 0.01% and 50%.

7. The photocatalyst of claim 1, wherein the WO₃ flower-like assembly has a particle size between 100 nm and 250 nm, and has a round shape, a spherical shape or a flower-like shape.

8. The photocatalyst of claim 1, wherein the WO₃ flower-like assembly is further doped with a metal ion, the metal ion being an ion of Ti, Zr, Mn, Fe, Pd, Pt, Cu, Ag, Zn, Al or Ce.

9. A disinfection material comprising the photocatalyst of claim 1.

10. A method for fabricating a photocatalyst comprising:
providing a modulator solution comprising a dicarboxylic acid or oxalic acid;
providing a sodium tungstate dihydrate solution;
mixing the sodium tungstate dihydrate solution and the modulator solution thereby forming a first mixture solution;
adding an acid into the first mixture solution thereby forming a second mixture solution;
heating the second mixture solution thereby forming a first precipitate;
collecting the first precipitate from the second mixture solution;
the first precipitate thereby forming WO₃ flower-like assemblies; and
doping the WO₃ flower-like assemblies with particles of a metal or a metal oxide such that the particles are distributed on the surface of each WO₃ flower-like assembly thereby forming the photocatalyst.

11. The method of claim 10, wherein said doping comprises mechanically mixing the WO₃ flower-like assemblies and a powder comprising the particles to distribute the particles on the surface of each WO₃ flower-like assembly.

12. The method of claim 10, wherein said doping comprises:
dispersing the WO₃ flower-like assemblies in a solution containing the particles to distribute the particles on the surface of each WO₃ flower-like assembly thereby forming second precipitate;
collecting the second precipitate from the solution; and
drying the second precipitates thereby forming the photocatalyst.

13. The method of claim 10, wherein the metal is Ti, Zr, Mn, Fe, Pd, Pt, Cu, Ag, Zn, Al or Ce.

14. The method claim 10, wherein the metal oxide is an oxide of Ti, Zr, Mn, Fe, Pd, Pt, Cu, Ag, Zn, Al or Ce.

15. The method claim 10, wherein the dicarboxylic acid is 1,4-benzendicarboxylic acid.

16. The method of claim 10, wherein the sodium tungstate dihydrate solution comprises sodium tungstate dihydrate in a concentration between 0.05 mol/L and 0.30 mol/L.

17. The method of claim 10, wherein the sodium tungstate dihydrate and oxalic acid in the first mixture solution are in a mass ratio between 1:1.1 and 1:2.0.

18. The method of claim 10, wherein the acid is hydrochloric acid (HCl).

19. The method of claim 18, wherein the HCl is added into the first mixture solution to adjust pH value of the second mixture solution to be 0.1 to 1.0.

20. The method of claim 10, wherein the second mixture solution is heated at 80-100°C for 8-12 hours.
